# FASCICULE DE BREVET EUROPEEN

(11) **EP 0 900 577 B1**
(45) Date de publication et mention de la délivrance du brevet: **05.04.2006**
(21) Numéro de dépôt: 98402173.3
(22) Date de dépôt: 02.09.1998
(51) Int. Cl.: A61N 1/375, H01R 13/52

(54) **Système autobloquant pour connecter une sonde à un générateur de stimulateur cardiaque, défibrillateur et/ou cardioverteur**
Selbstblockierendes System zum Anschliessen eines Herzleiters an einen Herzschrittmacher/Defibrillator/Kardioverter
Self-locking system to connect a cardiac lead to a pacemaker/defibrillator/cardioverter

(30) Priorité: 02.09.1997 FR 9710910
(43) Date de publication de la demande: 10.03.1999
(73) Titulaire: ELA MEDICAL (Société anonyme), 92541 Montrouge (FR)
(72) Inventeur: D'Hiver, Philippe, 92130 Issy Les Moulineaux (FR)
(74) Mandataire: Dupuis-Latour, Dominique

(56) Documents cités:
- EP-A- 0 357 941
- EP-A- 0 747 999
- DE-A- 4 010 181
- US-A- 4 913 147
- US-A- 5 069 209
- US-A- 5 082 453
- US-A- 5 086 773
- US-A- 5 252 090
- US-A- 5 275 620

## Description

La présente invention concerne les dispositifs médicaux implantables actifs. Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs comportent un boîtier généralement désigné "générateur" ou "générateur d'impulsions" raccordé électriquement et mécaniquement à une sonde, ce raccordement étant réalisé par le chirurgien au moment de l'implantation.

On pourra à cet effet se reporter à la norme française et européenne NF EN 50077 *"Connecteur à bas profil pour stimulateurs cardiaques implantables",* qui définit un système de connexion normalisé dit "IS-1" permettant de garantir l'interchangeabilité des sondes et des générateurs d'impulsions produits par différents fabricants (l'invention n'est toutefois pas limitée au cas particulier des systèmes de connexion selon cette norme, ni même aux systèmes de connexion pour stimulateurs cardiaques).

Jusqu'à présent, le raccordement entre le connecteur de la sonde et le connecteur du générateur était réalisé par une ou plusieurs vis, serrées par le chirurgien au moyen d'un outil *ad hoc* (tournevis muni éventuellement d'un limiteur de couple) au moment de l'implantation.

Ce raccordement par vis présente cependant plusieurs inconvénients. En premier lieu, outre un outil spécifique pour sa mise en oeuvre, cette technique impose la présence de bouchons de passage étanche de cet outil (pour éviter que, après implantation, les bornes de connexion ne viennent en contact avec des fluides organiques). Cette exigence d'étanchéité au niveau des fentes de passage de l'outil lors de la connexion implique un surcoût et une augmentation de volume du générateur au niveau du connecteur.

Par ailleurs, ce système de raccordement n'est pas à l'abri d'un oubli de serrage de vis de la part du chirurgien, d'un serrage insuffisant, ou d'un serrage trop violent endommageant le dispositif.

Le EP-A-0 747 999 propose, pour résoudre cette difficulté, un système de connecteur utilisant un empilement de disques déformables traversés axialement par le connecteur de la sonde, et configurés de manière à réaliser du seul fait de cet enfichage un contact électrique entre le connecteur de la sonde et les disques, qui sont conducteurs et reliés au connecteur du générateur.

Ce système, s'il évite le recours à des vis avec les divers inconvénients précités qui y sont liés, n'assure cependant pas la solidarisation mécanique entre sonde et générateur, de sorte que des moyens distincts doivent être prévus pour empêcher la séparation de ces deux éléments, par exemple suite à une traction inopinée exercée sur le câble de la sonde après enfichage du connecteur de cette dernière dans le connecteur du générateur.

Le US-A-5 069 209 propose un système de connecteur utilisant une douille de serrage pourvue de doigts flexibles exerçant ponctuellement un effort dirigé radialement sur le connecteur de soude. L'invention propose une autre structure de connecteur pour assurer un assujettisement fiable du connecteur de sonde au boîtier du générateur.

L'invention reste parfaitement compatible avec, notamment, le standard mécanique dimensionnel IS-1, ce qui lui permet d'accepter sans modification toutes les sondes normalisées actuelles ou à venir.

À cet effet, le dispositif médical implantable actif de l'invention, qui comprend un connecteur de générateur comportant un logement femelle axial recevant un connecteur de sonde et des moyens de retenue mécanique du connecteur de sonde dans le logement, ce dispositif comportant un organe élastique formant ledit moyen de retenue mécanique, cet organe élastique étant susceptible d'être librement déformé et mis sous tension élastique par déplacement, dans le sens de l'insertion, du connecteur de sonde dans le logement femelle axial, cette déformation libre étant non réversible de manière que l'organe élastique vienne exercer un effort de résistance, très supérieur à la force d'insertion, à l'encontre du déplacement du connecteur de sonde dans le sens du retrait, est

L'invention propose à cet effet un dispositif du type général décrit par le US-A-5 069 209 précité, correspondant au préambule de la revendication 1, et comprenant les éléments énoncés à la partie caractéristique de cette revendication. Les sous-revendications visent des mises en oeuvre préférentielles de l'invention.

D'autres caractéristiques de l'invention apparaîtront à la lecture de la description détaillée ci-dessous d'un exemple de réalisation.
Les figures 1a et 1b illustrent, respectivement en coupe longitudinale et en perspective, un premier mode de réalisation de l'invention.
Les figures 2a et 2b illustrent une variante du mode de réalisation des figures la et 1b.
Les figures 3a et 3b illustrent un deuxième mode de réalisation de l'invention, la figure 3c illustrant un outil de déblocage approprié.
Les figures 4a et 4b illustrent une troisième mode de réalisation de l'invention.

Sur les figures, la référence 10 désigne de façon générale l'extrémité proximale de la sonde (qui est indépendante du mode de réalisation de l'invention), sonde qui est par exemple du type bipolaire, notamment une sonde conforme au standard mécanique dimensionnel IS-1 mentionné plus haut. Une telle sonde comporte une première surface conductrice interne 12, protégée par une première gaine isolante 14 comportant par exemple des reliefs périphériques tels que 16 permettant d'assurer une fonction d'étanchéité, et une seconde surface conductrice 18, coaxiale à la première et décalée axialement, protégée par une seconde gaine 20, elle-même également munie de reliefs d'étanchéité 22.

L'extrémité 10 de la sonde est destinée à être insérée dans un connecteur de générateur comportant deux bornes de liaison destinées à coopérer avec les surfaces conductrices 12, 18. Une seule de ces bornes a été illustrée sur la figure, en 24, borne qui coopère avec la surface conductrice 18 de la sonde ; le générateur comporte une borne semblable, non représentée, destinée à coopérer avec la surface conductrice 12.

La borne 24 comporte un organe, caractéristique de l'invention, permettant d'assurer à la fois la liaison électrique avec la surface conductrice 18 et la retenue mécanique de la sonde pour empêcher sa déconnexion accidentelle.

Plus précisément, la borne selon l'invention permet, par simple enfichage, de réaliser un blocage de la sonde dans le connecteur par un effet d'arc-boutement ou de coincement, de sorte que l'effort de désinsertion éventuel (par exemple par une traction inopinée sur le câble de la sonde) soit très supérieur à l'effort d'insertion.

Alors que ce blocage est assuré automatiquement, sans outil, du seul fait de l'insertion et sans recours à une vis ou à un système de serrage analogue, le déblocage requerra en revanche l'utilisation d'un outil, d'où une sécurité accrue.

Par ailleurs, la borne de l'invention permet le blocage de pièces mâles cylindriques de révolution, qui est le cas le plus fréquent, mais, comme on le comprendra, peut également être appliquée le cas échéant au serrage de pièces à section polygonale.

En outre, bien que l'invention soit décrite dans le cadre d'une borne assurant simultanément la liaison électrique et le blocage mécanique, on peut dissocier ces deux fonctions, une borne selon l'invention servant au seul blocage mécanique de l'extrémité de la sonde dans le connecteur, et la liaison électrique étant assurée par un organe distinct, soit de type connu soit réalisé, lui aussi, selon les enseignements de l'invention.

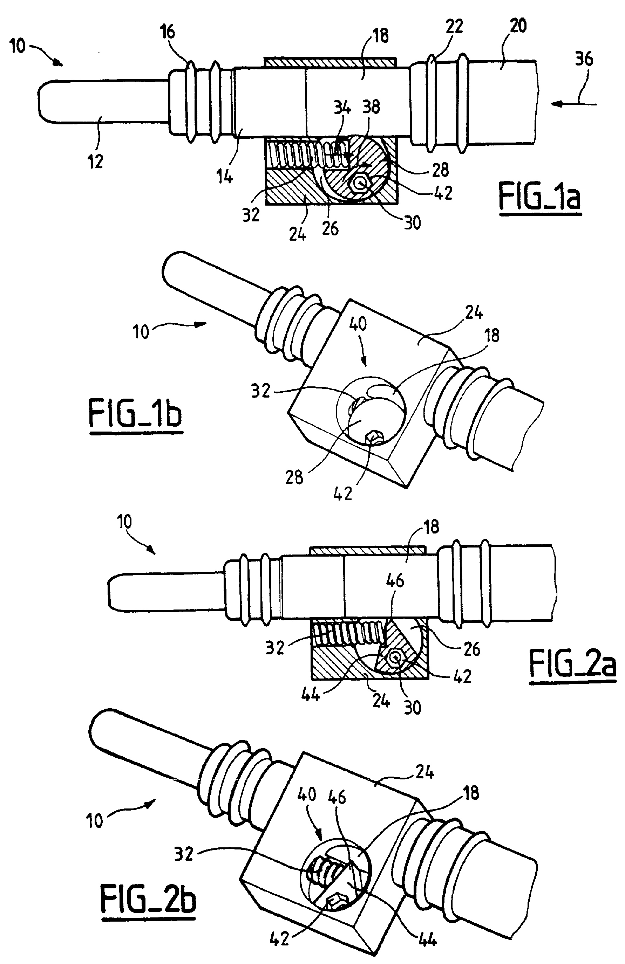

Les figures 1a et 1b illustrent un premier mode de réalisation de la borne 24 selon l'invention.

La borne 24 comporte un logement intérieur 26 recevant une pièce mobile 28 en forme de cylindre aplati, articulé en rotation autour d'un axe 30 décalé par rapport à l'axe central de la pièce 28, de manière à permettre à cette dernière de jouer un rôle d'excentrique ou de came (l'axe 30 peut être réel (articulation physique de la pièce 28) ou virtuel). Le logement 28 est placé dans la borne 24 en situation latérale, axialement au-delà de la surface conductrice 18.

La pièce mobile 28 est sollicitée en direction tangentielle par un ressort 32 dans le sens 34 inverse du sens 36 d'insertion de l'extrémité de sonde dans le connecteur ; cette sollicitation provoque une rotation de l'excentrique 28 autour de son axe 30, dans le sens de la flèche 38.

Ce dispositif fonctionne de la manière suivante.

Avant insertion de l'extrémité de sonde 10 dans la borne 24, la pièce 28, sollicitée par le ressort 34, fait légèrement saillie à l'intérieur du logement destiné à recevoir le connecteur de sonde. A la mise en place de l'extrémité de sonde dans le connecteur, sous l'effort d'insertion la pièce 28 s'escamote dans le logement 26 et laisse passer sans difficulté l'extrémité de la sonde. En revanche, lors d'une tentative de désinsertion de la sonde, la pièce 28 s'arc-boute, en cas d'axe virtuel, entre le fond du logement 26 et la surface 18 de l'extrémité de sonde 10 (cette surface 18 est une surface métallique rigide, à la différence des gaines 14 et 20 qui sont en matériau souple) ; en cas d'axe réel, l'arc-boutement se fait sur cet axe. L'ensemble est alors bloqué et peut résister à des efforts très supérieurs aux efforts d'insertion.

Pour renforcer la fonction de blocage, la surface de la pièce 28 en contact avec la surface conductrice 18 peut être éventuellement rendue rugueuse.

Pour débloquer le système et permettre le retrait de la fiche, la borne comporte une ouverture latérale 40 (fig. 1b) permettant d'accéder à une empreinte 42, par exemple hexagonale, formée sur la pièce 28 à l'endroit de l'axe de rotation 30. Il suffit alors à l'opérateur d'insérer dans cette empreinte 42 un outil, par exemple un simple tournevis à tête hexagonale, pour faire tourner la pièce 28 dans le sens contraire de la flèche 38, à l'encontre de la pression exercée par le ressort 32, jusqu'à ce que la pièce 28 ne fasse plus saillie à l'intérieur du logement recevant le connecteur de sonde. L'effort de déblocage avec l'outil est bien entendu très inférieur à l'effort de blocage en cas de tentative de désinsertion. Si nécessaire, une légère poussée de la sonde dans le sens de l'insertion facilite le déblocage de la pièce 28.

Les figures 2a et 2b sont homologues des figures 1a et 1b, dans une variante du premier mode de réalisation où la pièce 28 en forme de cylindre plat est remplacée par une pièce 44 avec une section en forme de secteur circulaire (au lieu de la section circulaire de la pièce 28 des figures 1a et 1b). Cette pièce 44 comporte ainsi, d'un côté, une région en forme de portion de cylindre venant en contact avec le fond du logement 26 pour permettre l'arc-boutement indiqué plus haut et, à l'opposé, une arête 46 permettant de concentrer la pression contre la surface conductrice 18 en cas d'arc-boutement et de renforcer ainsi le phénomène recherché, par effet de coin.

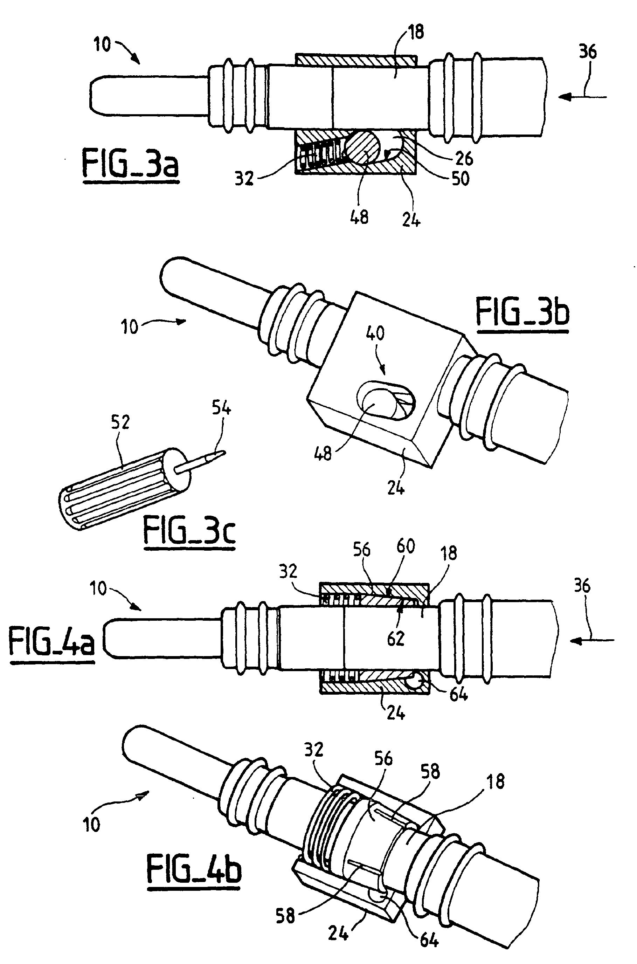

Dans un second mode de réalisation, illustré figure 3a et 3b, la pièce de blocage est une bille ou, comme représenté, un cylindre 48 mobile en translation dans le logement 26 guidé par une rampe 50 configurée de manière à rapprocher le cylindre 48 de l'axe de la sonde au fur à mesure de sa sollicitation dans le sens inverse du sens de l'insertion 36. Lorsque la bille est entièrement repoussée vers la gauche (avec les conventions de la figure), c'est-à-dire le ressort 32 comprimé au maximum, la bille s'escamote complètement dans le logement 26 sans faire saillie dans le logement du connecteur de sonde ; en revanche, lorsque le cylindre 48 est déplacé vers la droite (soit à vide, sous l'effet du ressort 32, soit en cas de tentative de désinsertion), le cylindre 48 tend à déborder du logement 26 jusque dans le logement du connecteur de sonde, venant ainsi bloquer ce dernier, s'il est présent, entre la surface conductrice 18 et la surface de la rampe 50 ;

Le déblocage est réalisé au moyen d'un outil 52 tel qu'illustré figure 3c, par exemple un simple pointeau comportant une extrémité conique 54. Cette extrémité est introduite dans l'ouverture 40 jusque dans le logement 26, et permet de repousser le cylindre 48, à l'encontre du ressort 32, jusque dans sa position complètement escamotée.

Les figures 4a et 4b illustrent un troisième mode de réalisation, dans lequel la pièce de blocage est une pièce mobile en translation, en forme de bague conique 56 coaxiale à l'élément conducteur 18, de même que le ressort de rappel 32 qui la sollicite.

La bague 56 comporte une surface conique 60 coopérant avec une surface conique inverse, homologue 62 de la borne 24. La combinaison des surfaces 60, 62 et des fentes 58 est telle que, lorsque la bague 56 est sollicitée dans le sens contraire du sens d'insertion 36 lors d'une tentative de désinsertion, elle vient pincer la surface conductrice 18 et s'opposer ainsi au retrait.

Pour le déblocage, on peut prévoir une lumière 64 dans laquelle fait saillie l'extrémité de la bague ; l'insertion d'un pointeau conique (outil du même type que celui illustré figure 3c) dans cette lumière 64 aura pour effet de repousser la bague à l'encontre de la sollicitation du ressort 32 et permettre ainsi le déblocage de l'ensemble.

## Revendications

1. Un dispositif médical implantable actif, notamment un stimulateur cardiaque, défibrillateur et/ou cardioverteur, comprenant un connecteur de générateur comportant un logement femelle axial recevant un connecteur de sonde (10) et des moyens de retenue mécanique du connecteur de sonde dans le logement, ce dispositif comportant un organe élastique (28, 32 ; 32, 44 ; 32, 48 ; 32, 56) formant ledit moyen de retenue mécanique, cet organe élastique étant susceptible d'être librement déformé et mis sous tension élastique par déplacer ment, dans le sens de l'insertion (36), du connecteur de sonde dans le logement femelle axial, cette déformation libre étant non réversible de manière que l'organe élastique vienne exercer un effort de résistance, très supérieur à la force d'insertion, à l'encontre du déplacement du connecteur de sonde dans le sens du retrait, l'organe élastique comportant une pièce mobile escamotable (28 ; 44 ; 48 ; 56) et un ressort de rappel (32) exerçant sur la pièce escamotable un effort de sollicitation pour amener cette dernière en contact avec le connecteur de sonde,
**caractérisé en ce que** ladite pièce mobile escamotable est une pièce rigide et **en ce que** ledit effort de sollicitation comport une composante orientée dans le sens contraire dudit sens de l'insertion.

2. Le dispositif de la revendication 1, dans lequel l'organe élastique est inclus dans une borne de liaison (24) comportant un moyen de mise en contact électrique propre à exercer une pression d'appui d'un élément conducteur de la borne contre un élément conducteur (18) du connecteur de sonde, l'organe élastique formant alors à la fois ledit moyen de retenue mécanique et ledit moyen de mise en contact.

3. Le dispositif de la revendication 1, dans lequel la pièce escamotable est une pièce mobile en rotation (28 ; 44).

4. Le dispositif de la revendication 1, dans lequel la pièce escamotable est une pièce mobile en translation (48 ; 56).

5. Le dispositif de la revendication 4, dans lequel la pièce escamotable est une bille ou un cylindre (48) mobile le long d'une rampe (50) inclinée par rapport à l'axe du logement axial.

6. Le dispositif de la revendication 4, dans lequel la pièce escamotable est une bague conique fendue (56) coopérant avec un profil conique homologue (62) du logement axial.

7. Le dispositif de la revendication 1, comprenant en outre des moyens de déverrouillage de l'organe élastique, pour exercer sur celui-ci, de l'extérieur, une sollicitation mécanique permettant de neutraliser ledit effort de résistance et permettre le retrait du connecteur de sonde hors de la borne de liaison.

## Patentansprüche

1. Aktive implantierbare medizinische Vorrichtung, insbesondere Herzschrittmacher, Defibrillator und/oder Kardioverter, mit einem Generatorverbinder, der eine einen Sondenverbinder (10) aufnehmende weibliche axiale Aufnahme und Mittel zum mechanischen Rückhalten des Sondenverbinders in der Aufnahme umfasst, wobei die Vorrichtung ein elastisches Element (28, 32; 32, 44; 32, 48; 32, 56) umfasst, welches das mechanische Rückhaltemittel bildet, wobei das elastische Element geeignet ist, durch Versetzung, in Einführrichtung (36), des Sondenverbinders in der weiblichen axialen Aufnahme frei verformt und unter elastische Spannung gesetzt zu werden, wobei diese freie Verformung nicht umkehrbar ist, so dass das elastische Element eine als die Einführkraft viel höhere Widerstandskraft entgegen die Versetzung des Sondenverbinders in Rückführrichtung ausübt, wobei das elastische Element ein bewegliches versenkbares Teil (28; 44; 48; 56) und eine Rückholfeder (32) aufweist, die auf das versenkbare Teil eine Betätigungskraft ausübt, um letzteres mit dem Sondenverbinder in Berührung zu bringen,
**dadurch gekennzeichnet, dass** das bewegliche versenkbare Teil ein starres Teil ist, und **dadurch**, dass die Betätigungskraft eine Komponente aufweist, die entgegen die Einführrichtung gerichtet ist.

2. Vorrichtung nach Anspruch 1, in welcher das elastische Element in einem Verbindungsanschluss (24) enthalten ist, der ein Mittel zur elektrischen Verbindung aufweist, das geeignet ist, einen Anpressdruck eines leitenden Elements des Anschlusses gegen ein leitendes Element (18) des Sondenverbinders auszuüben, wobei das elastische Element dann sowohl das mechanische Rückhaltemittel als auch das in Berührung bringende Mittel bildet.

3. Vorrichtung nach Anspruch 1, in welcher das versenkbare Teil ein drehbar bewegliches Teil ist (28; 44).

4. Vorrichtung nach Anspruch 1, in welcher das versenkbare Teil ein geradlinig bewegliches Teil ist (48; 56).

5. Vorrichtung nach Anspruch 4, in welcher das versenkbare Teil eine Kugel oder ein Zylinder (48) ist, die/der entlang einer im Verhältnis zur Achse der axialen Aufnahme schrägen Rampe (50) beweglich ist.

6. Vorrichtung nach Anspruch 4, in welcher das versenkbare Teil ein geschlitzter konischer Ring (56) ist, der mit einem entsprechenden konischen Profil (62) der axialen Aufnahme zusammenwirkt.

7. Vorrichtung nach Anspruch 1, außerdem mit Mitteln zur Entriegelung des elastischen Elements, um auf dieses von außen eine mechanische Betätigung auszuüben, die es erlaubt, die Widerstandskraft aufzuheben, und um eine Rückführung des Sondenverbinders aus dem Verbindungsanschluss zu erlauben.

## Claims

1. An active implantable medical device, in particular a cardiac pacemaker, defibrillator and/or cardioverter, including a generator connector comprising an axial female recess receiving a lead connector (10) and means for mechanically securing the lead connector in the recess, said device including an elastic member (28, 32 ; 32, 44 ; 32, 48 ; 32, 56) forming said mechanically securing means, said elastic member being liable to be freely deformed and elastically tensioned by displacing, in the direction of insertion (36), the lead connector in the axial female recess, said free deformation being non-reversible so that the elastic member may exert a resisting effort, much greater than the insertion force, against the displacement of the lead connector in the direction of withdrawal, the elastic member including a retractable movable part (28 ; 44 ; 48 ; 56) and a return spring (32) exerting upon the retractable part a biasing effort for bringing the latter in contact with the lead connector,
**characterized in that** said retractable movable part is a rigid part and **in that** said biasing effort has a component directed in a direction opposite to said direction of insertion.

2. The device of claim 1, wherein the elastic member is included in a coupling terminal (24) comprising an electric contacting means suitable for exerting an effective pressure by a conductive element of the terminal against a conductive element (18) of the lead connector, the elastic member then forming both said mechanically securing means and said contacting means.

3. The device of claim 1, wherein the retractable part is a rotatably mov able part (28 ; 44).

4. The device of claim 1, wherein the retractable part is a translationally movable part (48 ; 56).

5. The device of claim 4, wherein the retractable part is a ball or cylinder (48) movable along a ramp (50) slanted with respect to the axial recess.

6. The device of claim 4, wherein the retractable part is a slit conical ring (56) co-operating with a corresponding conical profile (62) of the axial recess.

7. The device of claim 1, further comprising means for unlocking the elastic member, for exerting on the fatter, from the outside, a mechanical bias enabling to counter said resisting effort and enable withdrawal of the lead connector out of the coupling terminal.
